# EUROPÄISCHE PATENTSCHRIFT

(11) **EP 1 285 678 B1**
(45) Veröffentlichungstag und Bekanntmachung des Hinweises auf die Patenterteilung: **20.07.2005**
(21) Anmeldenummer: 02090297.9
(22) Anmeldetag: 21.08.2002
(51) Int. Cl.: A61N 1/05

(54) **Einzel-Elektrodensonde für Herzschrittmachersysteme**
Single electrode lead for pacemaker systems
Sonde à électrode unique pour système de stimulation cardiaque

(30) Priorität: 23.08.2001 DE 10142834
(43) Veröffentlichungstag der Anmeldung: 26.02.2003
(73) Patentinhaber: BIOTRONIK Mess- und Therapiegeräte GmbH & Co Ingenieurbüro Berlin, 12359 Berlin (DE)
(72) Erfinder: Taskiran, Murat, 91052 Erlangen (TR); Flach, Erhard, 12305 Berlin (DE); Schaldach, Max, - (DE)
(74) Vertreter: Eisenführ, Speiser & Partner

(56) Entgegenhaltungen:
- EP-A- 0 009 732
- EP-A- 0 779 079
- EP-A- 0 788 808
- WO-A-00/76570
- FR-A- 2 713 492
- US-A- 4 154 247
- US-A- 5 769 077
- US-A- 5 782 239

## Beschreibung

Nach Erkenntnissen der Herzschrittmachertechnik wird bei einschlägigen pathologischen Befunden die Anwendung von so genannten DDD-Herzschrittmachersystemen empfohlen. Diese Spezifikation "DDD" (= Dual pacing/Dual sensing/Demand + Triggered) bedeutet, dass einerseits eine Stimulation ("Pacing") in beiden Herzkammern vorgenommen wird und dass andererseits noch vorhandene Eigenaktionen des Sinus- bzw. AV-Knotens des Herzens sowohl im Atrium als auch im Ventrikel erfasst werden ("Sensing"). Zu diesem Zweck sind bereits so genannte "Double-Lead"-DDD-Schrittmachersysteme bekannt. Deren Hauptnachteile ist es jedoch, dass zwei getrennte Elektrodensonden in das Herz implantiert und dort positioniert werden müssen.

Es sind ferner Einzelsonden bekannt, bei denen eine ventrikulare Elektrode an der Sondenspitze und zwei atrialen Ringelektroden in entsprechender Entfernung von der Ventrikelelektrode am Sondenkörper angeordnet sind. Die atrialen Ringelektroden werden dazu verwendet, das atriale Potential zu erfassen, wodurch die ventrikulare Stimulation nach einer geeigneten atrioventrikularen Verzögerung getriggert wird. Die ventrikuläre Stimulation kann bipolar erfolgen. Grundsätzlich ist es zwar denkbar, die vorstehend erörterte Einzel-Elektrodensonde auch für DDD-Herzschrittmachersysteme einzusetzen. Jedoch tritt hier ein Problem auf, dass die atrialen Ringelektroden nicht wandständig platziert sind, sondern frei in Blutstrom schwimmen Diese sogenannten "flottierenden" Elektroden im Atrium führen zu erheblichen Einschränkungen der Stimulationseigenschaften, da meist sehr hohe Stimulationsamplituden benutzt werden müssen. Der Hauptgrund hierfür ist die fehlende Wandstandigkeit der flottierenden Elektroden. Die hohen Stimulationsamplituden bringen den Nachteil eines hohen Energieverbrauches und häufiger Phrenikus-Stimulationen mit sich.

EP 0 779 079 offenbart demgegenüber eine Einzel-Elektrodensonde (Single-Lead), der auf ihrer im implantierten Zustand im Vorhof platzierten Teillange eine solche Form gegeben wird, dass mindestens eine atriale Elektrode - üblicherweise eine Ringelektrode - der Sonde an der Vorhofwand des Herzens wandstandig anlegbar ist Um dies zu erreichen, ist die Sonde auf der genannten Teillänge mit einem elastischen Vorformelement vorgesehen, welches der Sonde dort eine definiert ausgelenkte Form gibt. Auf Grund seiner Elastizitat kann das Vorformelement jedoch aus seiner definierten Gestalt in einen im Wesentlichen geradlinigen gestreckten Zustand übergeführt werden, was mithilfe des üblichen, bei der Sondenimplantation verwendeten Führungsdraht erfolgt. Der Führungsdraht muss dabei in seinen Elastizitatseigenschaften natürlich signifikant steifer sein als das Vorformelement.

Beim Implantationsvorgang wird vor der eigentlichen lmplantation der Sonde der Führungsdraht bis zum Anschlag an die Sondenspitze vorgeschoben, wobei durch diesen Vorschub des Fuhrungsdraht eine Zugkraft auf den Sondenkorper ausgeübt wird, die zu einer Streckung der Sonde im Bereich des Vorformelementes führt. In diesem gestreckten Zustand kann dann die Sonde in das Herz eingeführt werden. Nach der Implantierung der Sonde und dem Herausziehen des Führungsdrahtes wird die Sonde dann auf der besagten Teillänge durch das Vorformelement so ausgelegt, dass die Sonde mit mindestens einer atrialen Ringelektrode an der Vorhofwand des Herzens anliegt. Nachteilig hierbei erweist sich die relativ komplizierte Ausführung dieser Einzel-Elektrodensonde. Ferner ist eine Verwendung eines Führungsdrahtes bei der Implantation der Einzel-Elektrodensonde nicht wünschenswert.

Aus EP 0 426 089 ist eine Elektrodenanordnung mit zwei getrennten Elektrodensonden bekannt, wobei eine der Elektrodensonden in das Atrium und weiter ins Ventrikel eingeführt wird, während die andere Elektrodensonde als Gegenelektrode außen am Herzen, d. h. als endocardiale Gegenelektrode, angebracht wird. Am distalen Ende der Elektrodenleitung ist eine Helix-Elektrode 25 als Spitzenelektrode vorgesehen, die in dem rechten ventrikulären Myokard befestigt wird, indem sie dort eingeschraubt wird. Oberhalb der Spitzenelektrode ist ein Abschnitt der Leitung zweigeteilt ausgeführt, wobei beide Stränge des geteilten Abschnittes jeweils eine Wendelelektrode 22, 24 zur Defibrillation aufweisen. Die beiden Verzweigungen sind gegeneinander vorgespannt, so dass sie im implantierten Zustand voneinander beabstandet angeordnet sind. Die Elektroden 22, 24 sollen dabei einen Kontakt mit dem rechten ventrikulären Myokard bilden. Die zweigeteilte Elektrodenleitung und deren Elektroden 22, 24 wurden hier gewahlt, um mit den endocardialen Elektroden 18, 20 ein vierseitiges Polygon (orthogonal electrode placement) zu bilden. Nachteilig ist hierbei jedoch, dass zwei getrennte Elektrodensonden implantiert werden müssen. Ferner ist die Elektrodenleitung nur im Ventrikel ausreichend befestigt, so dass die sich im Atrium befindlichen Elektroden als flottierende Elektroden ausgebildet sind. Ein elektrischer Kontakt der Spritzenelektrode im Ventrikel kann ohne weiteres sichergestellt werden, wahrend der elektrische Kontakte im Atrium kritisch sein kann und somit die oben beschriebenen Nachteile aufweist. Ferner sind für die Befestigung von Elektroden im Atrium andere Voraussetzungen gegeben als zur Befestigung von Elektroden im Ventrikel, da es sich bei dem Atrium im Wesentlichen um einen "Durchgang" handelt.

Außerdem zeigt eine Figur 4f der US 4,154,247 eine Elektrodenleitung mit einem verzweigten Abschnitt 620, der der Abbildung gemäß zur Platzierung im Ventrikel vorgesehen ist, der gemäß der Beschreibung aber auch im Atrium des Herzens platziert sein kann. Die US 4,154,247 sagt jedoch nichts zur Fixierung der Elektrodenleitung.

Das Dokument EP-A-0 009 732 offenbart die Einzel-Elektrodensonde, die als nächstliegender Stand der Technik betrachtet wird.

Aufgabe der Erfindung ist es, eine im Atrium eines Herzens zu fixierenden Elektrodenleitung anzugeben.

Erfindungsgemäß wird diese Aufgabe durch eine Einzel-Elektrodensonde gemäß Anspruch 1 gelöst, bei der der zweigeteilte Abschnitt auf der Elektrodenleitung angeordnet und ausgestaltet ist, der Elektrodenleitung im implantierten Zustand Halt im Atrium eines Herzens zu verschaffen.

Ein derartig zweigeteilter Abschnitt der Elektrodenleitung trägt wesentlich zur Verbesserung des Haltes von Elektroden am atrialen Myokard bei, ohne dabei die Ausgestaltung der Elektrodenleitung wesentlich zu komplizieren.

Bei der Erfindung sind jeweils zwei Ringelektroden an den jeweiligen Strängen des zweigeteilten Abschnittes der Elektrodenleitung angeordnet.

Bei einer bevorzugten Ausführungsform der Erfindung verlaufen die beiden Stränge des zweigeteilten Abschnittes der Elektrodenleitung parallel zueinander und sind derart vorgeformt, dass sie sich konvex nach außen wolben, um im implantierten Zustand diametral gegenüberliegend mitsamt der Ringelektroden gegen das atriale Myokard gedrückt zu werden.

Eine weitere Ausführungsvariante besteht in einer Elektrodenleitung, die eine versteifende Wendel aus elastischem Material besitzt, welche in einer Vielzahl von Windungen gebildet wird. Im Lumen dieser Elektrodenleitung ist eine Sehne vorgesehen, die mit ihrem distalen Ende an der Elektrodenleitung befestigt ist. Durch Zug an der Sehne ergibt sich auf diese Weise eine stauchende Kraft in der Elektrodenleitung. Die Wendel ist so ausgebildet, dass die Elektrodenleitung bei Zug auf der Sehne aus ihrer Längsrichtung ausbiegt und eine vorgegebene dreidimensionale Form annimmt. Beim Einsatz entsprechender Materialien kann diese Form fixiert werden, so dass die Elektrodenleitung die dreidimensionale Verformung beibehält, wenn der Zug über die Sehne nachlässt. Eine derartige Ausgestaltung der Elektrodenleitung kann alternativ oder zusätzlich zum vorgenannten Vorformen der Elektrodenleitung vorgesehen sein. Zum Fixieren der Sehne unter Zug kann beispielsweise im Bereich eines Elektrodensteckers am proximalen Ende der Elektrodenleitung eine Klammerung oder Crimpung vorgesehen sein. Die Sehne wird dementsprechend nach der Implantation der Elektrodenleitung angezogen oder gestrafft um im angezogenen Zustand im Bereich des Elektrodensteckers fixiert. Mit einer derartigen Elektrodenleitung lassen sich großere Krümmungskrafte erzielen, als mit einer lediglich vorgeformten Elektrodenleitung.

Eine andere Ausführungsform zeichnet sich durch ein Memorymetallelement im zu verformenden Bereich der Elektrodenleitung aus, welches beispielsweise eine an sich bekannte Titanlegierung aufweist, die bei Überschreiten einer Auslösetemperatur ihre Form von einer ersten zu einer zweiten Form verändert. Dieses Memorymetallelement ist so ausgeführt, dass seine erste Form einer im Wesentlichen gestreckten Elektrodenleitung entspricht, die ein einfaches Einfuhren der Elektrodenleitung erlaubt, während die zweite Form des Memorymetalls nach Uberschreiten der Auslosetemperatur zu einer erfindungsgemäß verformten Elektrodenleitung führt. Vorteilhafter Weise kann ein Heizelement zum Erwärmen des Memorymetallelementes auf die Auslösetemperatur vorgesehen sein, falls die Körpertemperatur nicht ausreicht, um die Auslösetemperatur zu erreichen. Falls die Körpertemperatur ausreicht, um die Auslösetemperatur zu erreichen, bei der das Memorymetall seine Form verändert, können Kühlmittel vorgesehen sein, alternativ können die Elektrodenleitungen auch im gekühlten Zustand eingeführt werden, so dass es sich während und nach dem Einführen in das Blutgefäß langsam erwärmt und schließlich die Auslosetemperatur erreicht.

Die Erfindung soll nun anhand von Ausführungsbeispielen mithilfe der Figuren näher erläutert werden. Diese zeigen:
- Figur 1: ein zum Platzieren im Herzen vorgesehenes distales Ende einer Elektrodenleitung;
- Figur 2: der in Figur 1 abgebildete Abschnitt der Elektrodenleitung platziert in einem Herzen.
- Figuren 3a bis d: ein Ausführungsdetail einer Elektrodenleitung die durch Stauchen einer versteifenden Wendel verformbar ist;

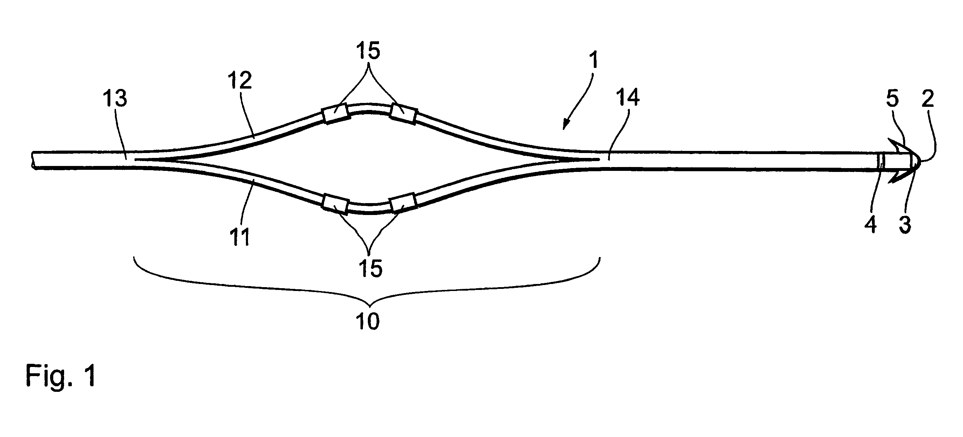

Figur 1 zeigt jenen Abschnitt einer Single-Lead-Elektrodenleitung 1, welcher zum Platzieren in einem Herzen gedacht ist. Ein distales Ende 2 der Elektrodenleitung 1 sowie der unmittelbar daran anschließende Abschnitt der Elektrodenleitung 1 sind zum Platzieren in einem Ventrikel eines Herzens vorgesehen. Dazu weist die Elektrodenleitung 1 eine Spitzenelektrode 3, eine in der Nähe der Spitzenelektrode befindliche Ringelektrode 4 und so genannte Tines 5 zum Verankern der Elektrodenleitung im Myokard des Ventrikels auf.

An den zum Platzieren im Ventrikel vorgesehen, distalen Abschnitt der Elektrodenleitung 1 schließt sich ein Abschnitt 10 an, der zum Platzieren im Atrium eines Herzens vorgesehen ist. Im Bereich des Abschnitts 10 ist die Elektrodenleitung 1 in zwei Stränge 11 und 12 aufgeteilt. Die beiden Stränge 11 und 12 vereinigen sich an einem proximalen Ende 13 des zweigeteilten Abschnitts 10 zu einer einzigen Elektrodenleitung, die beispielsweise zu einem implantierbaren Schrittmacher führt. Am distalen Ende 14 des zweigeteilten Abschnitts 10 vereinigen sich die beiden Stränge 11 und 12 zu jenem distalen Abschnitt der Elektrodenleitung 1, der für die Anordnung im Ventrikel ausgebildet ist.

Die beiden Stränge 11 und 12 tragen jeweils zwei Ringelektroden 15, die der Stimulation des Myokards im Bereich des Atriums dienen sollen.

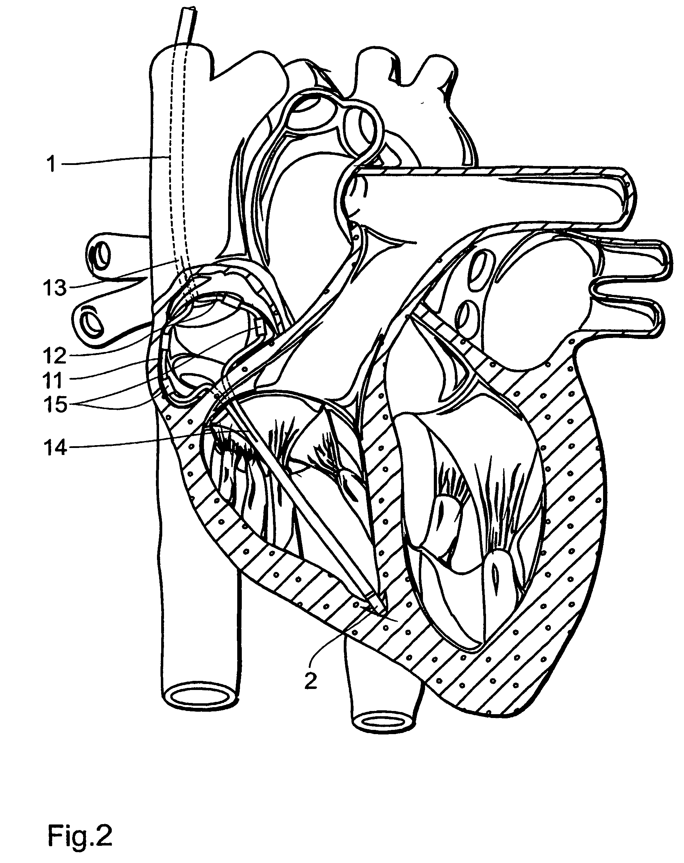

Die beiden Stränge 11 und 12 sind derart gegeneinander vorgespannt, dass sie im implantierten Zustand insbesondere im Bereich der Elektroden 15 diametral einander gegenüberliegende Wände des Atriums gedrückt werden, wie dies in Figur 2 dargestellt ist. Die beiden Stränge 11 und 12 der Elektrodenleitung 1 sind im implantierten Zustand - abgesehen von den Übergangsbereichen bei 13 und 14 - konvex nach außen gewölbt, um einen guten elektrischen Kontakt der Ringelektroden 15 zum atrialen Myokard sicherzustellen und gleichzeitig die Elektrodenleitung 1 im Atrium allein durch die Vorformung zu fixieren.

Im Gegensatz zu den Befestigungsmöglichkeiten einer Elektrodenleitung im Ventrikel, welche beispielsweise anhand einer Spitzenelektrode an dem ventrikulären Myokard befestigt werden kann, ist dies im Atrium nicht moglich, da es sich bei dem Atrium im Wesentlichen um einen "Durchgang" handelt. Eine Befestigung an dem atrialen Myokard ist mittels einer Spitzenelektrode nicht möglich, da die Elektrodenleitung lediglich durch das Atrium in das Ventrikel eingeführt wird.

Die gute Haltefunktion des zweigeteilten Abschnittes der Elektrodenleitung am atrialen Myokard und damit ein guter elektrischer Kontakt der sich auf den beiden Strängen 11 und 12 befindlichen Ringelektroden 15 mit dem atrialen Myokard wird durch die nach außen gewölbten Stränge 11 und 12 realisiert, welche an den diametral gegenüberliegenden Punkten im Atrium auf Grund ihrer gegenseitigen Vorspannung aufgespannt sind. Die Elastizitatseigenschaften der Strange 11 und 12 sind dabei derart gewählt, dass sichergestellt wird, dass der elektrische Kontakt der Elektroden 15 mit dem atrialen Myokard dauerhaft gewährleistet wird.

Alternativ zu dem vorstehend beschriebenen Ausführungsbeispiel kann der zweigeteilte Abschnitt auch derart ausgestaltet werden, dass nur an einem der beiden Strange 11 und 12 eine oder mehrere Elektroden 15 vorgesehen werden.

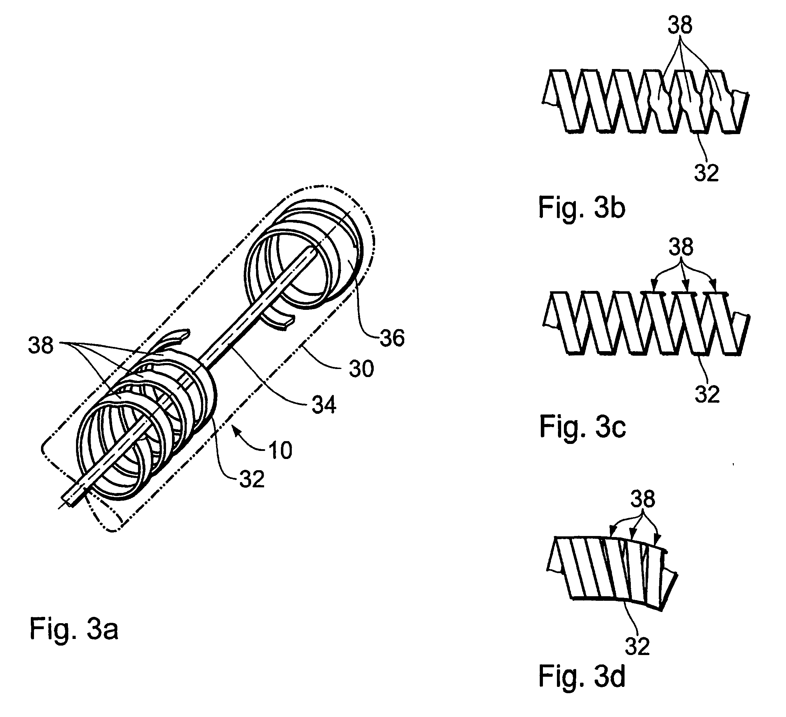

Die Figuren 3a bis d zeigen in einer Detaildarstellung eine Ausführungsvariante der Elektrodenleitung, die in gestreckter Form in das Atrium des Herzens eingeführt werden kann und nach dem Einführen ihre erfindungsgemaße Form annehmen kann.

Der Elektrodenleitungsabschnitt 10 in Figur 3 umfasst eine Hülle 30 (in Figur 3a nur angedeutet) sowie innerhalb der Hülle 30 eine Metallwendel 32 und eine in einem von der Metallwendel 32 eingeschlossenen Lumen angeordnete Sehne 34 die an ihrem distalen Ende über eine Verbindungsplatte 36 mit der Metallwendel 32 verbunden ist.

Die Figur 3b zeigt einen Abschnitt der Metallwendel 32 in der Draufsicht; die Figur 3c den Abschnitt der Metallwendel 32 in der Seitenansicht. Zu erkennen ist, dass die einzelnen Windungen der Metallwendel 32 voneinander beabstandet sind und dass das Bandmaterial, aus dem die Metallwendel 32 besteht, an den Stellen 38 jeweils breiter ausgeführt ist. Durch Zug auf die Sehne 34 verkürzt sich die Metallwendel 32 bis die Windungen der Metallwendel 32 aneinander anliegen; siehe Figur 3d. Da das Bandmaterial der Metallwendel 32 an den Stellen 38 jeweils verbreitert ist, behält die verkürzte oder gestauchte Metallwendel 32 nicht ihre längsgestreckte Form, sondern nimmt die in Figur 3d dargestellte Biegung an Nach dem in Figur 3 dargestellten Prinzip können die Wendeln entsprechend der Metallwendel 32 so gestaltet werden, dass eine Elektrodenleitung durch Zug auf eine eingebrachte Sehne beliebige dreidimensionale Krümmungen annimmt. Ohne Zug auf die Sehne ist die Elektrodenleitung gestreckt und biegeweich und kann leicht in das Blutgefäß eingeführt werden, wie dies in Figur 3a angedeutet ist.

Eine Einzel-Elektrodensonde wie die dargestellte besitzt üblicherweise einen (hier nicht abgebildeten) Elektrodenstecker an ihrem proximalen Ende, um die Elektrodenleitung mit einem Therapiegerät wie einem Herzschrittmacher zu verbinden. Zum Fixieren der Sehne unter Zug kann beispielsweise im Bereich eines solchen Elektrodensteckers eine Klammerung oder Crimpung vorgesehen sein. Der Elektrodenstecker weist entsprechend vorzugsweise eine Klammer- oder Crimpeinrichtung auf. Die Sehne wird dementsprechend nach der Implantation der Elektrodenleitung angezogen oder gestrafft und im angezogenen Zustand im Bereich des Elektrodensteckers fixiert.

## Patentansprüche

1. Einzel-Elektrodensonde für DDD- Herzschrittmachersysteme mit nur einer Elektrodenleitung, wobei die Einzel-Elektrodensonde eine Elektrodenleitung aufweist, welche einen zweigeteilten Abschnitt (10) aufweist, an dessen Beginn (13) sich ein Elektrodenleitungsstrang in zwei Stränge (11, 12) aufteilt, welche sich am Ende (14) des Abschnittes (10) wieder zu einem Strang vereinen, wobei der zweigeteilte Abschnitt auf der Elektrodenleitung (1) der Einzel-Elektrodensonde angeordnet und gestaltet ist, der Elektrodenleitung der Einzel-Elektrodensonde im implantierten Zustand Halt im Atrium eines Herzens zu verschaffen, wobei jeweils zwei Ringelektroden (15) zur Abgabe elektrischer Energie an angrenzendes Myokard an den jeweiligen Strängen (11, 12) des zweigeteilten Abschnittes der Elektrodenleitung (1) der Einzel-Elektrodensonde angeordnet sind.

2. Einzel-Elektrodensonde nach Anspruch 1, bei der die beiden Stränge (11, 12) des zweigeteilten Abschnitts (10) der Elektrodenleitung (1) der Einzel-Elektrodensonde parallel zu einander verlaufen und derart vorgeformt sind, dass sie sich im implantierten Zustand konvex nach außen wölben, um im implantierten Zustand diametral gegenüberliegend mitsamt der Ringelektroden (15) gegen das atriale Myokard gedrückt zu werden.

3. Einzel-Elektrodensonde nach Anspruch 1, bei der die Elektrodenleitung der Einzel-Elektrodensonde eine Hülle aufweist, die eine versteifende Wendel (32) aus elastischem Material einschließt, die von einer Vielzahl von Windungen gebildet ist und ihrerseits ein Lumen einschließt, **dadurch gekennzeichnet, dass** im Lumen der Elektrodenleitung der Einzel-Elektrodensonde eine Sehne (34) derart angeordnet und mit einem distalen Ende der Sehne (34) befestigt ist, dass in der Elektrodenleitung (1) der Einzel-Elektrodensonde eine in Längsrichtung der Elektrodenleitung (1) wirkende, die zumindest den Abschnitt (10) stauchende Kraft erzeugbar ist und dass die Windungen der Wendel (32) bildende elastische Material derart geformt ist, dass sich die Elektrodenleitung der Einzel-Elektrodensonde bei angespannter Sehne (34) und Wirken der stauchenden Kraft dreidimensional verformt.

4. Einzel-Elektrodensonde nach Anspruch 1, bei der die Elektrodenleitung der Einzel-Elektrodensonde ein Memorymetallelement aufweist welches seine Form bei überschreiten einer Sprungtemperatur von einer ersten vorgegebenen Form zu einer zweiten vorgegebenen Form verändert, wobei die erste Form des Memorymetallelementes mit einer im Wesentlichen gestreckten Elektrodenleitung der Einzel-Elektrodensonde korrespondiert und die zweite Form zu einer dreidimensional verformten Elektrodenleitung der Einzel-Elektrodensonde führt.

## Claims

1. Single electrode probe for DDD cardiac pacemaker systems, the probe having only one electrode line, wherein the single electrode probe comprises an electrode line comprising a bifurcated section (10), one electrode line portion separating into two portions (11, 12) at the beginning (13) of the bifurcated section, which portions rejoin at an end (14) of the section (10), wherein the bifurcated section is arranged and configured on the electrode line (1) of the single electrode probe to hold the electrode line of the single electrode probe in the atrium of a heart when in an implanted condition, wherein for the delivery of electrical energy to adjoining myocardium two respective ring electrodes (15) are arranged on the respective portions (11, 12) of the bifurcated section of the electrode line (1) of the single electrode probe.

2. Single electrode probe according to claim 1, wherein the two portions (11, 12) of the bifurcated section (10) of the electrode line (1) of the single electrode probe extend in mutually parallel relationship and are pre-shaped in such a way that they are curved convexly outwardly in the implanted condition in order in the implanted condition to be pressed in diametrically-opposed relationship together with the ring electrodes (15) against the atrial myocardium.

3. Single electrode probe according to claim 1, wherein the electrode line of the single electrode probe comprises a casing which encloses a stiffening coil (32) of elastic material which is formed by a large number of turns and which in turn encloses a lumen, **characterised in that** a cord (34) is arranged in the lumen of the electrode line of the single electrode probe and is fixed with a distal end of the cord (34), such that a force which acts in the longitudinal direction of the electrode line (1) and which upsets at least the section (10) can be produced in the electrode line (1) of the single electrode probe, and **in that** the elastic material forming the turns of the coil (32) is shaped in such a way that the electrode line (1) of the single electrode probe is three-dimensionally deformed when the cord (34) is tightened and the upsetting force is operative.

4. Single electrode probe according to claim 1, wherein the electrode line of the single electrode probe comprises a memory metal element which changes from a first predetermined shape to a second predetermined shape when a jump temperature is exceeded, wherein the first shape of the memory metal element corresponds to a substantially stretched electrode line of the single electrode probe and the second shape results in a three-dimensionally deformed electrode line of the single electrode probe.

## Revendications

1. Sonde électrode unique pour systèmes de stimulateurs cardiaques type DDD ("dual sensing/dual pacing/demand+triggered" ou double chambre) comportant seulement une ligne d'électrode, la sonde électrode unique présentant une ligne d'électrode qui présente un tronçon (10) en deux parties, au début (13) duquel un conducteur de ligne d'électrode se divise en deux conducteurs (11, 12) qui se réunissent à la fin (14) du tronçon (10) pour former de nouveau un conducteur, le tronçon divisé en deux de la sonde électrode unique étant agencé sur la ligne d'électrode (1) et configuré de manière à procurer à la ligne d'électrode de la sonde électrode unique, à l'état implanté, un soutien dans l'atrium d'un coeur, deux électrodes annulaires (15) étant agencées sur les conducteurs respectifs (11, 12) du tronçon divisé en deux de la ligne d'électrode de la sonde électrode unique, pour fournir de l'énergie électrique au myocarde adjacent.

2. Sonde électrode unique selon la revendication 1, dans laquelle les deux conducteurs (11, 12) du tronçon (10) divisé en deux de la ligne d'électrode (1) de la sonde électrode unique s'étendent parallèlement l'un à l'autre et sont préformés de telle sorte qu'à l'état implanté, ils sont bombés vers l'extérieur sous forme convexe pour, à l'état implanté, être pressés avec les électrodes annulaires (15) contre le myocarde atrial, en étant diamétralement opposés l'un à l'autre.

3. Sonde électrode unique selon la revendication 1, dans laquelle la ligne d'électrode de la sonde électrode unique présente une enveloppe qui renferme un filament spiralé (32) rigidifiant en matériau élastique qui est formé par une multitude de spires et qui renferme à son tour un lumen, **caractérisé en ce que** dans le lumen de la ligne d'électrode de la sonde électrode unique est agencée une fibre (34) qui est fixée par son extrémité distale de telle sorte que dans la ligne d'électrode (1) de la sonde électrode unique peut être produite une force agissant en direction longitudinale de la ligne d'électrode (1) et qui comprime au moins le tronçon (10), et **en ce que** le matériau élastique formant les spires du filament spiralé (32) est déformé de telle sorte que la ligne d'électrode (1) de la sonde électrode unique est déformée en trois dimensions lorsque la fibre (34) est tendue et lorsque la force de compression agit.

4. Sonde électrode unique selon la revendication 1, dans laquelle la ligne d'électrode de la sonde électrode unique présente un élément métallique à mémoire qui, en cas de dépassement d'une température de transition, modifie sa forme à partir d'une première forme prédéterminée pour atteindre une deuxième forme prédéterminée, la première forme de l'élément métallique à mémoire correspondant à une ligne d'électrode de la sonde électrode unique sensiblement en extension et la deuxième forme menant à une ligne d'électrode de la sonde électrode unique déformée sur trois dimensions.
